# EUROPEAN PATENT SPECIFICATION

(11) **EP 1 370 223 B1**
(45) Date of publication and mention of the grant of the patent: **22.12.2010**
(21) Application number: 02728520.4
(22) Date of filing: 19.03.2002
(51) Int. Cl.: A61K 8/22, A61K 8/44, A61Q 5/10

(54) **OXIDIZING COMPOSITIONS COMPRISING A CHELANT AND A CONDITIONING AGENT AND METHODS OF TREATING HAIR**
OXIDIERENDE ZUSAMMENSETZUNGEN, DIE EINEN KOMPLEXBILDNER UND EIN KONDITIONIERMITTEL ENTHALTEN, UND METHODEN ZUR HAARBEHANDLUNG
COMPOSITIONS OXYDANTES COMPRENANT UN AGENT CHELATANT ET UN AGENT REVITALISANT ET PROCEDES DE TRAITEMENT DES CHEVEUX

(30) Priority: 20.03.2001 GB 0106946; 30.11.2001 GB 0128750
(43) Date of publication of application: 17.12.2003
(73) Proprietor: THE PROCTER & GAMBLE COMPANY, Cincinnati, OH 45202 (US)
(72) Inventor: MCKELVEY, Graham, Neil, St. John's Woking, Surrey GU21 1RQ (GB); MARSH, Jennifer, Mary, Henley-on-Thames, Oxfordshire RG9 1XL (GB); DUNBAR, James, Charles, Morrow, OH 45152 (US)
(74) Representative: Kohol, Sonia
(86) International application number: PCT/US2002/008483
(87) International publication number: WO 2002/074273

(56) References cited:
- EP-A- 1 001 011
- WO-A-94/16672
- WO-A-97/24106
- DE-A- 4 404 177
- US-A- 6 024 891

## Description

### FIELD

The present invention relates to oxidizing compositions comprising a chelant and a conditioning agent and to methods of treating hair. The compositions and methods disclosed provide enhanced deposition of conditioning agents on hair subjected to oxidative treatments such as bleaching, oxidative hair dyeing and perming.

### BACKGROUND

Melanin is a natural pigment found in hair. Melanin and hair-forming cells are naturally produced in the hair bulb at the root of the hair. As new cells are produced, the older ones are pushed upwards out of the skin to form the hair shaft, which is the part of the hair that can be seen above the scalp. Hair can be schematically described as being made of a center part called the cortex, which contains the melanin, and an outer layer called the cuticle. It is the cortex that gives hair its special qualities such as elasticity and curl.

The hair shaft is made of dead cells that have turned into a mixture of different forms of the special hair protein, keratin. Keratin contains high concentrations of a particular amino acid called cystine. Every cystine unit contains two cysteine amino acids in different chains, which have come to lie near each other and are linked together by two sulphur atoms, forming a very strong chemical bond known as a disulphide linkage. This cross-linking by disulphide linkages between the keratin chains accounts for much of the strength of the hair.

Bleaching and dyeing (or coloring) of hair has become increasingly popular over the past years. Younger people may want to change the natural color of their hair to a more fashionable one, while older people may also use dyeing compositions to conceal gray hair. As people grow older, the production of melanin slows, giving more and more gray hair over time. Melanin can be purposely altered by chemical treatments to give lighter shades. The lightening is achieved by oxidizing the melanin pigments, usually with an oxidizing agent in alkaline solution, also called bleaches. Examples of oxidizing agents that can be used are hydrogen peroxide, potassium, sodium or ammonium salts of perborate or percarbonate, persulfate and percarbamide.

Bleaches are also used during oxidative dyeing treatments. Oxidative (or "permanent") dye compositions comprise "precursor dyes" which are small molecules capable of diffusing into the hair. These molecules mainly belong to three classes of aromatic compounds: diamines, aminophenols and phenols. They are sufficiently small to diffuse in the hair shaft where, once activated by an oxidizing agent such as hydrogen peroxide, they further react with other precursors to form larger colored complexes. Oxidative hair dye compositions commonly contain, in addition to the dye precursors and a source of peroxide, a variety of additional cosmetic and peroxide stabilizing agents.

Oxidizing agents can activate oxidative dye precursors across a range of pH. However, it is known that enhanced dye oxidation can be achieved via the use of a hair-swelling agent (HSA) that can adjust the pH of the oxidizing solution. Such HSA's further enhance the oxidizing and dyeing process by swelling the hair fibers to aid both the diffusion of the peroxide and dyeing agents into the hair and enabling faster, more thorough dye oxidization and hair dyeing. Preferred hair-swelling agents for adjusting the pH of peroxide hair oxidizing compositions are aqueous alkaline solutions containing ammonia (ammonium hydroxide) or monoethanolamine(MEA).

Low levels of chelants are routinely used as stabilizers or preservatives in various oxidizing compositions. For example, EDTA (ethylenediaminetetraacetic acid) is commonly used as a stabilizer in hydrogen peroxide solution, which would otherwise decompose too rapidly and could not be stored for a long time. Ethylene diaminedissucinnic acid (EDDS) is also known as a good stabilizing agent component to increase the stability of laundry bleaching products. Amounts as low as 0.1% by weight of the oxidizing composition are usually used to stabilize the oxidizing agent contained in said oxidizing compositions.

US6024891 describes a method to improve the stability of a bleach and lipophile composition comprising an aminoalkylsilicone. Chelating agents are described to aid cleaning and bleach stability.

WO97/24106 describes hair coloring compositions comprising a peroxygen bleach, a organic peroxyacid bleach precursor and hair colouring agents. Chelants are described to sequester heavy metal ions.

EP1001011 describes bleaching compostions comprising a peroxybleach and alkoxylated benzoic acid to provide chemical stability particularly on storage. Cheating agents are described to increase stain removal and bleaching.

WO94/03553 discloses peroxygen bleaching composition stabilised with EDDS.

Oxidative treatments of hair such as bleaching (decoloration) and oxidative dyeing give good results and are very commonly used. They are however not without drawbacks. The oxidizing agents used for bleaching and oxidative dyeing damage hair to some extent. The mechanism by which damage is caused to the hair fibers is not perfectly understood. However, it is known that some of the disulphide bonds linking the keratin chains break in the presence of oxidizing compositions. Repeated oxidative treatments leave weak, brittle hairs, which have little shine and luster. An enormous effort has been made to address this problem over the past years, and various solutions have been proposed.

US4,138,478 discloses agents for reducing the damage to hair during bleaching and dyeing by the use of a water-soluble 3-amino-1-hydroxypropane-1,1-diphosphonic compound for protecting hair from damage by "nascent oxygen". According to this patent, "the diphosphonic compound is substantively adsorbed by the hair and acts to hinder degradation of the hair by nascent oxygen which is either present therewith or which is substantially added". Other protective compounds such as hydroxyethane-1,1 diphosphonic acid (HEDP) and ethylenediaminetetramethylene phosphonic acid (EDTMP) are disclosed at low levels in US3,202,579 and US3,542,918.

Today, most dyeing or bleaching compositions are sold with a conditioner that is applied on hair after the bleaching or dyeing composition has been rinsed off and/or contain a conditioning agents. Examples of conditioning agents are silicones, cationic surfactants and cationic polymers. Conditioners do not bring the hair back to its initial condition but conceal the damage under a protective layer of the conditioning agent, which results in an improved feel of the hair.

It has now been surprisingly found that chelants, when formulated in levels higher than suggested in the prior art, substantially increase the deposition of conditioning agent (or "conditioners") on hair during or after an oxidative treatment such as bleaching or oxidative dyeing, resulting in longer-lasting improved hair feel. It has been found that for this purpose the chelants can be applied to hair either at the same time as the oxidizing composition or as a pretreatment. Chelants, conditioning agents and oxidizing compositions are preferably applied simultaneously.

Without being bound by theory, it is believed that the mechanism of deposition is enhanced for two reasons. Firstly, chelants are believed to trap very efficiently environmental and intrinsic heavy metal ions such as iron, manganese and copper. It is believed that in the absence of chelants, these heavy metal ions react with hydrogen peroxide and form free radicals that weaken the structure of hair by destroying the disulphide bonds of keratin. It is furthermore believed that non-cationic conditioning agents such as silicones deposit less efficiently on damaged hair and therefore chelants, by reducing oxidative hair damage, substantively increase the efficiency of the deposition of the conditioners.

Secondly, it is believed that when chelants and conditioning agents are used simultaneously, the high levels of chelants required to carry out the present invention considerably increases the ionic strength of the composition (most chelants are in the form of at least partially dissolved salts at the preferred pH of from about 8 to about 12) which drives an increased deposition of the conditioning agents on hair.

### SUMMARY

The present invention is directed to a composition comprising, by weight of the composition:
a) an oxidizing agent;
b) at least 1.6% of a chelant;
c) a conditioning agent selected from silicone materials, especially nonvolatile silicone and amino functionalised silicones, cationic surfactants, cationic polymers, alkoxylated amines and mixtures thereof, and at least one oxidative dye precursor

The present invention is also directed to a method of treating hair comprising the step of contacting hair with a composition according to the above definition.

The present invention is also directed to a method of treating hair comprising the steps of:
i) contacting hair with a first composition comprising at least 1.6% of a chelant and an oxidizing agent; and at least one oxidative dye precursor; and a
ii) contacting hair immediately after step i) with a second composition comprising a conditioning agent selected from silicone materials, especially nonvolatile silicone and amino functionalised silicones, cationic surfactants, cationic polymers, alkoxylated amines and mixtures thereof.

The present invention is further directed to kits adapted for treating hair according to these methods.

These and other features, aspects, and advantages of the present invention will become evident to those skilled in the art from a reading of the present disclosure.

### DETAILED DESCRIPTION

While the specification concludes with claims which particularly point out and distinctly claim the invention, it is believed the present invention will be better understood from the following description.

As used herein the term "hair" to be treated may be "living" i.e. on a living body or may be "non-living" i.e. in a wig, hairpiece or other aggregation of non-living keratinous fibers. Mammalian, preferably human hair is preferred. However wool, fur and other keratin containing fibers are suitable substrates for the compositions according to the present invention.

As used herein, the term "oxidizing composition" means a composition comprising at least one oxidizing agent suitable for use on hair, such as hydrogen peroxide, sodium, potassium, ammonium or other salts of perborate, percarbonate, persulfate and percarbamide. Non-limiting examples of such compositions are oxidative dye compositions and bleaching compositions.

As used herein the term "oxidative treatment of hair" or "hair treatment comprising at least one oxidative step" is used in the broad sense in that it is intended to encompass all treatments of hair comprising at least one step of contacting hair with at least one oxidizing composition. Preferred examples of oxidative treatment for human hair are bleaching, oxidative dyeing or perming.

As used herein the term "immediately" means within about 1 hour, preferably within about 30mn, more preferably within about 15mn.

All percentages are by weight of the total composition unless specifically stated otherwise. When more than one composition are used during a treatment, the total weight to be considered is the total weight of all the compositions applied on hair simultaneously (i.e. the weight found "on head") unless otherwise specified. All ratios are weight ratios unless specifically stated otherwise.

All cited references are incorporated herein by reference in their entireties. Citation of any reference is not an admission regarding any determination as to its availability as prior art to the claimed invention.

### Chelants

The term "chelant" (or "chelating agent" or "sequestering agent") is well known in the art and refers to a molecule or a mixture of different molecules each capable of forming a chelate with a metal ion. A chelate is an inorganic complex in which a compound (chelant) is coordinated to a metal ion at two or more points so that there is a ring of atoms including the metals. Chelants contain two or more electron donor atoms that form the coordination bonds with the metal ion.

Any chelants is suitable for use herein. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996)

When related to chelants, the terms "salts thereof" mean all salts comprising the same functional structure as the chelant they are referring to (same chemical backbone) and having similar or better chelating properties. These terms include alkali metal, alkaline earth, ammonium, substituted ammonium salts (e.g monoethanolammonium, diethanolammonium, triethanolammonium),

Preferred chelants for use herein are carboxylic acids (in particular aminocarboxylic acids), phosphonic acids (in particular aminophosphonic acids) and polyphosphoric acids (in particular linear polyphosphoric acids), and their salts.

### Aminocarboxylic acids chelants

Carboxylic acid chelants as defined herein are chelants having at least one carboxylic acid moiety (-COOH).

Examples of aminocarboxylic acid chelants suitable for use herein include nitrilotriacetic acid and polyaminocarboxylic acids such as diethylenetriamine pentaacetic acid (DTPA), ethylenediamine disuccinic acid (EDDS), ethylenediamine diglutaric acid (EDGA), 2-hydroxypropylenediamine disuccinic acid (HPDS), glycinamide-N,N'-disuccinic acid (GADS), ethylenediamine-N-N'-diglutaric acid (EDDG), 2-hydroxypropylenediamine-N-N'-disuccinic acid (HPDDS), ethylenediaminetetraacetic acid (EDTA), salts thereof .

Other suitable aminocarboxylic type chelants for use herein are iminodiacetic acid derivatives such as N-2-hydroxyethyl N,N diacetic acid or glyceryl imino diacetic acid (described in EP-A-317,542 and EP-A-399,133), iminodiacetic acid-N-2-hydroxypropyl sulfonic acid and aspartic acid N-carboxymethyl N-2-hydroxypropyl-3-sulfonic acid (described in EP-A-516,102), β-alanine-N,N'-diacetic acid, aspartic acid-N,N'-diacetic acid, aspartic acid-N-monoacetic acid and iminodisuccinic acid chelants (described in EP-A-509,382), ethanoldiglycine acid, salts thereof .

EP-A-476,257 describes suitable amino based chelants. EP-A-510,331 describes suitable chelants derived from collagen, keratin or casein. EP-A-528,859 describes a suitable alkyl iminodiacetic acid chelants. Dipicolinic acid and 2-phosphonobutane-1,2,4-tricarboxylic acid are also suitable.

Preferred amino carboxylic chelants include diamirae-N,N'-dipolyacid and monoamine monoamide-N,N'-dipolyacid chelants and salts thereof . Preferred polyacids contain at least two acid groups independently selected from the carboxyl group (-COOH), the o-hydroxyphenyl group, the m-hydroxyphenyl group and the p-hydroxyphenyl group. Preferably at least one acid moeity is a carboxylic moiety. Suitable polyacids include diacids, triacids and tetraacids, preferably diacids. Preferred salts include alkali metal, alkaline earth, ammonium or substituted ammonium salts. EDTA is a tetramonoacid and does not belong to this class of preferred chelants.

Preferred aminocarboxylic chelants are diamine-N,N'-dipolyacid and monoamine monoamide-N,N'-dipolyacid chelants and salts thereof and . Preferred polyacids contain at least two acid groups independently selected from the carboxylic acid group (-COOH), sulphonic group (-SO₃H), the o-hydroxyphenyl group, the m-hydroxyphenyl group and the p-hydroxyphenyl group. Suitable polyacids include diacids, triacids and tetraacids, preferably diacids. Preferred salts include alkali metal, alkaline earth, ammonium or substituted ammonium salts. EDTA is a tetramonoacid and does not belong to this class of preferred chelants.

Preferably, the polyacids are di-carboxylic acids, preferably di-carboxylic acids having a carbon chain length of from about 3 to about 10 carbon atoms, more preferably from about 4 to about 6 carbon atoms, even more preferably about 4 carbon atoms.

Exemplary diamine dipolyacids suitable for use herein include ethylenediamine-N,N'-disuccinic acid (EDDS), ethylenediamine-N,N'-diglutaric acid (EDDG), 2-hydroxypropylenediamine-N,N'-disuccinic acid (HPDDS), all disclosed in European Patent EP0687292, ethylenedicysteic acid (EDC) disclosed in US5,693,854, diaminoalkyldi(sulfosuccinic acids) (DDS) disclosed in US5,472.642 and EDDHA (ethylenediamine-N-N'-bis(ortho-hydroxyphenyl acetic acid)), a method of preparation of which is disclosed in EP331556. A preferred monoamine monoamide-N,N'-dipolyacid is glycinamide-N,N'-disuccinic acid (GADS), described in US 4,983,315.

Highly preferred for use herein is ethylenediamine-N,N'-disuccinic acid (EDDS), and salts thereof. Preferred EDDS compounds for use herein are the free acid form, and salts thereof. Preferred salts include alkali metal, alkaline earth metals, ammonium and substituted ammonium salts (e.g. monoethanolammonium, diethanolammonium, triethanolammonium). Highly preferred salts are sodium, potassium, magnesium and calcium salts. Examples of such preferred sodium salts of EDDS include Na₂EDDS and Na₃EDDS.

The structure of the acid form of EDDS is as follows:

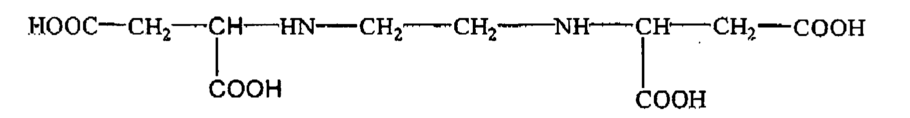

EDDS can be synthesised, for example, from readily available, inexpensive starting materials such as maleic anhydride and ethylenediamine. The synthesis of EDDS from maleic anhydride and ethylene diamine yields a mixture of three optical isomers, [R,R], [S,S], and [S,R] (25% S,S, 50% R,S and 25% R,R) , due to the two asymmetric carbon atoms. The biodegradation of EDDS is optical isomer-specific, with the [S,S] isomer degrading most rapidly and extensively.

US5,747,440, Kellett et al., discloses EDDS derivatives comprising an modified polyamine having units of the formula:

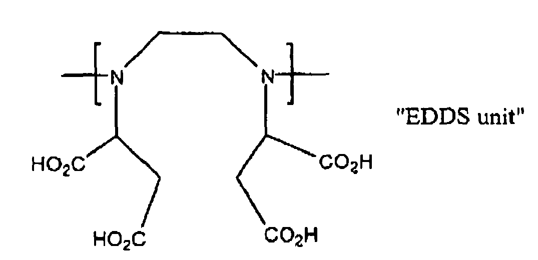

Preferred chelants that are not diamine-N,N'-dipolyacid and monoamine monoamide-N,N'-dipolyacid chelants include N,N'-bis(2-hydroxybenzyl)ethylenediamine-N,N'-diacetic acid (HBED) salts thereof and thereof: 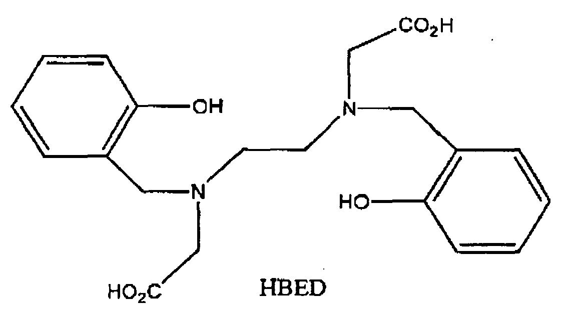 Examples of suitable HBED can be found in WO9744313, assigned to Novartis.

### Polyphosphoric acid chelants

Suitable polyphosphoric acid chelants include molecules which contain more than one P atom and have P-O-P bonds. Polyphosphoric acid chelants and salts (polyphosphates) can be linear and are generally represented by the formula [PₙO₃ₙ₊₁]⁽ⁿ⁺²⁾⁻M₍ₙ₊₂₎⁺ wherein M is a suitable counter-ion such as H⁺, Na⁺ or K⁺ and n an integer. Polyphosphoric acid type chelants and their polyphosphate salts can also be cyclic and have the formula [PₙO₃ₙ]ⁿ⁻Mₙ⁺. Representative examples include, among other, sodium tripolyphosphate tetrasodium diphosphates, hexametaphosphoric acid and sodium metaphosphate.

### Phosphonic acid chelants

Suitable phosphonic acid chelants include amino alkylene poly (alkylene phosphonic acid), ethane 1-hydroxy diphosphonic acids and nitrilo trimethylene phosphonic acids and salts thereof. Suitable chelants of this type are disclosed in US 4,138,478, Reese et al., US 3,202,579 and US3,542,918 .

Preferred phosphonic acid type chelants for use herein have the formula (I) below: 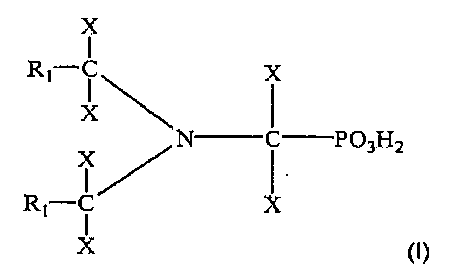 wherein each X are independently selected from hydrogen or alkyl radicals, preferably hydrogen or alkyl radicals having from 1 to 4 carbon atoms, preferably hydrogen; and each R₁ are independently selected from -PO₃H₂ or a group having the formula (II) below:

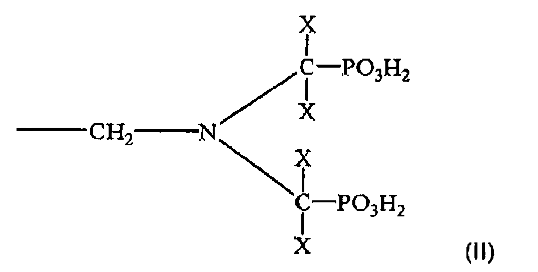

Preferred chelants according to Formula (I) for use herein are aminotri-(1-ethylphosphonic acid), ethylenediaminetetra-(1-ethylphosphonic acid), aminotri-(1-propylphosphonic acid), aminotri-(isopropylphosphonic acid) and chelants having the formula (III) below: 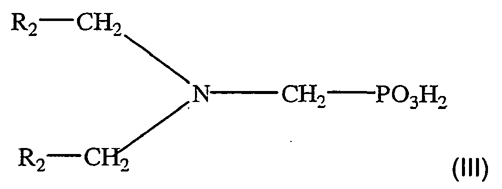 wherein each R₂ are independently selected from -PO₃H₂ or a group having the formula (IV) below:

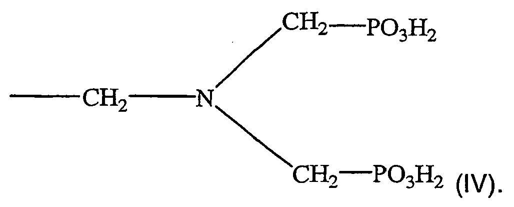

Especially preferred chelants according to formula (IV) for use herein are aminotri-(methylenephosphonic acid), ethylene-diamine-tetra-(methylenephosphonic acid) (EDTMP) and diethylene-triamine-penta-(methylenephosphonic acid) (DTPMP).

### Examples of other chelants:

Examples of other chelants suitable for use herein include but are not limited to polyethyleneimines as disclosed in US5,955,415.

### Levels

When used simultaneously with an oxidizing agent, chelants usually give good benefits above about 1.6% by weight of the composition. For most chelants the best results were achieved at levels of above about 2% by weight. Levels above 5% can also be used but it would not be practical to use such high amounts for regulatory and safety reasons. Preferred range is therefore of from above about 2% to about 5%, more preferably of from above about 2% to about 4%.

When used as a pretreatment (first composition) prior to the oxidizing step, chelants usually give good benefit at levels above 0.1%, preferably above 0.25%, more preferably above 0.5%, even more preferably above 1% by weight of said first composition used for the pretreatment (referred to in the claims and herein as "first composition"). For most chelants the best results were achieved at levels of about 2% by weight of the first composition.

When the conditioning agent is applied to hair as a post treatment after a first composition comprising a chelant and an oxidizing agent, said chelant is preferably present in the first composition at levels above 0.1 %, preferably above 0.25%, more preferably above 0.5%, even more preferably above 1% by weight of said first composition. Levels above 5% can also be used but it would not be practical to use such high amounts for regulatory and safety reasons. Preferred range is therefore of from above about 2% to about 5%, more preferably of from above about 2% to about 4%.

### Conditioning agent

The compositions of the present invention comprise or are used in combination with a composition comprising a conditioning agent. Conditioning agents for use herein are selected from silicone materials, especially nonvolatile silicone and amino functionalised silicones, cationic surfactants, cationic polymers, alkoxylated amines and mixtures thereof.

The conditioning agent will generally be used at levels of from about 0.05% to about 20% by weight of the composition, preferably of from about 0.1 % to about 15%, more preferably of from about 0.2% to about 10%, even more preferably of from about 0.2% to about 2%. The minimum level that is used in a particular composition should be effective to provide a conditioning benefit. The maximum level that can be used is not limited by theory, but rather by practicality. It is generally unnecessary and expensive to use levels in excess of about 10% and, depending on the type of agent (polymeric conditioners being most prone), such high levels can cause an undesirable weighting down of the hair.

When used as post-treatment immediately after the oxidizing composition has been applied and optionally rinsed off, the levels of conditioner in the post treatment composition are similar as those disclosed above, but by weight of the composition applied as a post-treatment.

Suitable silicone conditioning agents for use herein include nonvolatile and insoluble compound that intermix in the composition so as to be in the form of an emulsion, i.e., a separate, discontinuous phase of dispersed, insoluble droplets, usually suspended with a suspending agent. This dispersed silicone conditioning component will comprise a silicone fluid hair conditioning agent such as a silicone fluid and can also comprise other ingredients, such as a silicone resin to enhance silicone fluid deposition efficiency or, for example, enhance glossiness of the hair (especially when high refractive index (e.g. above about 1.46) silicone conditioning agents are used (e.g. highly phenylated silicones).

### Silicone materials

The silicone conditioning agent phase may comprise volatile silicone components. Typically, if volatile silicones are present, it will be incidental to their use as a solvent or carrier for commercially available forms of nonvolatile silicone materials ingredients, such as silicone gums and resins.

The silicone conditioning agent component for use herein will preferably have viscosity of from about 2.10⁻⁵ m²/s to about 2 m²/s (from about 20 to about 2,000,000 centistokes) at 25°C, more preferably from about 10⁻³ m²/s to about 1.8 m²/s (from about 1,000 to about 1,800,000 cSt), even more preferably from about 5.10⁻² m²/s to about 1.5 m²/s (from about 50,000 to about 1,500,000 cSt), most preferably from about 10⁻¹ m²/s to about 1.5 m²/s (from about 100,000 to about 1,500,000 cSt). The viscosity can be measured by means of a glass capillary viscometer as set forth in Dow Corning Corporate Test Method CTM0004, July 20, 1970.

One type of silicone fluid that can be used herein is a silicone oil. The term "silicone oil" shall mean flowable silicone materials having a viscosity of less than 1 m²/s (1,000,000 centistokes) at 25°C. Generally, the viscosity of the fluid will be between about 5.10⁻⁶m²/s and 1 m²/s (between about 5 and 1,000,000 centistokes) at 25°C, preferably between about 10⁻⁵m²/s and 0.1 m²/s (between about 10 and about 100,000 cSt). Suitable silicone oils include polyalkyl siloxanes, polyaryl siloxanes, polyalkylaryl siloxanes, polyether siloxane copolymers, and mixtures thereof. Other insoluble, nonvolatile silicone fluids having hair conditioning properties can also be used. More particularly silicone oils hereof include polyalkyl or polyaryl siloxanes having the general formula: 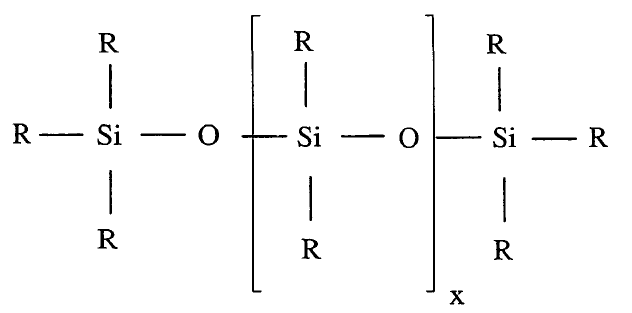 wherein R is aliphatic, preferably alkyl or alkenyl, or aryl, substituted or unsubstituted radical, and x is an integer from 1 to about 8,000. Suitable unsubstituted R groups include alkoxy, aryloxy, alkaryl, arylalkyl, arylalkenyl, alkamino, and ether-substituted, hydroxyl-substituted, and halogen-substituted aliphatic and aryl groups. Suitable R groups also include cationic amines and quaternary ammonium groups.

The aliphatic or aryl groups substituted on the siloxane chain may have any structure as long as the resulting silicones remain fluid at room temperature, are hydrophobic, are neither irritating, toxic nor otherwise harmful when applied to the hair and / or skin, are compatible with the other components of the composition, are chemically stable under normal use and storage conditions, are insoluble in the composition, and are capable of being deposited on and, of conditioning, the hair and / or skin. The two R groups on the silicon atom of each monomeric silicone unit may represent the same group or different groups. Preferably, the two R groups represent the same group.

Preferred alkyl and alkenyl substituents are C₁-C₅ alkyls and alkenyls, more preferably from C₁-C₄, most preferably from C₁-C₂. The aliphatic portions of other alkyl-, alkenyl-, or alkynyl-containing groups (such as alkoxy, alkaryl, and alkamino) can be straight or branched chains and preferably have from one to five carbon atoms, more preferably from one to four carbon atoms, even more preferably from one to three carbon atoms, most preferably from one to two carbon atoms. As discussed above, the R substituents hereof can also contain amino functionalities, e.g. alkamino groups, which can be primary, secondary or tertiary amines or quaternary ammonium. These include mono-, di- and trialkylamino and alkoxyamino groups wherein the aliphatic portion chain length is preferably as described above. The R substituents can also be substituted with other groups, such as halogens (e.g. chloride, fluoride, and bromide), halogenated aliphatic or aryl groups, and hydroxy (e.g. hydroxy substituted aliphatic groups). Suitable halogenated R groups could include, for example, trihalogenated (preferably fluoro) alkyl groups such as -R¹-C(F)₃, wherein R¹ is C₁-C₃ alkyl. Examples of such polysiloxanes include polymethyl -3,3,3 trifluoropropylsiloxane.

The nonvolatile polyalkylsiloxane fluids that may be used include, for example, polydimethylsiloxanes. These siloxanes are available, for example, from the General Electric Company in their Viscasil R and SF 96 series, and from Dow Corning in their Dow Corning 200 series. Also suitable for use herein is Dow Corning DC 1664 (TN) 60,000 cstk polydimethyl siloxane with 300nm particle size which is preferably used in combination with a deposition aid. Polydimethyl silicone is also known as dimethicone oil. Other suitable R groups include methyl, methoxy, ethoxy, propoxy, and aryloxy. The three R groups on the end caps of the silicone may also represent the same or different groups. Suitable R groups include methyl, ethyl, propyl, phenyl, methylphenyl and phenylmethyl. The preferred silicones are polydimethyl siloxane, polydiethylsiloxane, and polymethylphenylsiloxane. Polydimethylsiloxane is especially preferred.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid.

Other suitable silicone conditioning agents are disclosed in WO9804237 p.22-p.29 incorporated herein by reference.

The polyether siloxane copolymers that may be used include, for example, a polypropylene oxide modified polydimethylsiloxane (e.g., Dow Corning DC-1248) although ethylene oxide or mixtures of ethylene oxide and propylene oxide may also be used. The ethylene oxide and polypropylene oxide level must be sufficiently low to prevent solubility in water and the composition hereof. Alkylamino substituted silicones that can be used herein include those of the formula: 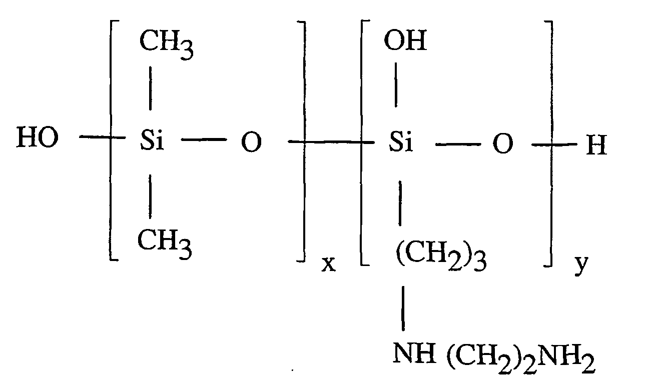 in which x and y are integers which depend on the molecular weight, the average molecular weight being approximately between 5,000 and 10,000. This polymer is also known as "amodimethicone". Other aminosilicones suitable for use herein are available from the General Electric Company under the trade names SF1703, SM2115, SM2101, and SM2658.

### Cationic surfactants

Cationic surfactants useful in compositions of the present invention contain amino or quaternary ammonium hydrophilic moieties which are positively charged when dissolved in the aqueous composition of the present invention. Cationic surfactants containing quaternary ammonium moieties and silicone moieties can also be employed. Cationic surfactants among those useful herein are disclosed in the following documents : M.C. Publishing Co., McCutcheon's, Detergents & Emulsifiers, (North American edition 1979); Schwartz, et al., Surface Active Agents, Their Chemistry and Technology, New York: Interscience Publishers, 1949; U.S. Patent 3,155,591, Hilfer, issued November 3, 1964; U.S. Patent 3,929,678, Laughlin, et al., issued December 30, 1975; U.S. Patent 3,959,461, Bailey, et al., issued May 25, 1976; and U.S. Patent 4,387,090, Bolich, Jr., issued June 7, 1983.

Among the quaternary ammonium-containing cationic surfactant materials useful herein are those of the general formula: 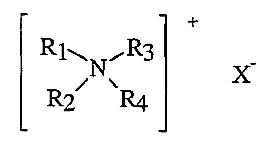 wherein R₁-R₄ are independently an aliphatic group of from about 1 to about 22 carbon atoms, aryl, or an alkoxy, polyoxyalkylene, alkylamido, hydroxyalkyl, or alkylaryl group having from about 1 to about 22 carbon atoms; and X is an anion selected from halogen (especially chlorine), acetate, phosphate, nitrate and alkylsulfate (preferably C₁-C₃ alkyl) radicals. The aliphatic groups may contain, in addition to carbon and hydrogen atoms, ether linkages, and other groups such as amino groups. The longer chain aliphatic groups, e.g., those of about 12 carbons, or higher, can be saturated or unsaturated.

Quaternary ammonium salts include dialkyldimethyl-ammonium chlorides, wherein the alkyl groups have from about 12 to about 22 carbon atoms and are derived from long-chain fatty acids, such as hydrogenated tallow fatty acid (tallow fatty acids yield quaternary compounds wherein R₁ and R₂ have predominately from 16 to 18 carbon atoms). Other cationic surfactants include those wherein at least one of the R₁-R₄ radicals contains one or more hydrophilic moieties selected from alkoxy (preferably C₁-C₃ alkoxy), polyoxyalkylene (Preferably C₁-C₃ polyoxyalkylene), alkylamido, hydroxyalkyl, alkylester, and combinations thereof. Optionally, the cationic conditioning surfactant contains from 2 to about 10 nonionic hydrophile moieties in the R₁-R₄ groups. For purposes herein, each hydrophilic amido, alkoxy, hydroxyalkyl, alkylester, alkylamido or other unit is considered to be a distinct nonionic hydrophile moiety.

Other cationic surfactants useful herein are disclosed in WO9632919.

### Cationic polymers

Suitable cationic polymers are disclosed in WO9632919 p.17-21. Examples of cationic polysaccharide polymer materials suitable for use herein include those of the formula: 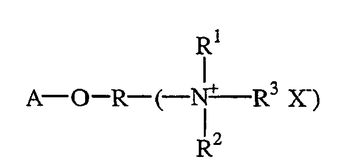 wherein:
A is an anhydroglucose residual group, such as a starch or cellulose anhydroglucose residual,
R is an alkylene oxyalkylene, polyoxyalkylene, or hydroxyalkylene group, or combination thereof,
R¹, R², and R³ independently are alkyl, aryl, alkyl, aryl, arylalkyl, alkoxyalkyl, or alkoxyaryl groups, each group containing up to about 18 carbon atoms, and the total number of carbon atoms for each cationic moiety (i.e., the sum of carbon atoms in R¹, R² and R³) preferably being about 20 or less, and
X is an anionic counterion, as previously described.

Cationic cellulose is available from Amerchol Corp. (Edison, NJ, USA) in their Polymer JR^{TN}, LR^{TN}, KG^{TN} and LK^{TN} series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200 (or Quatrisoft).

### Alkoxylated amines

Alkoxylated amines are conditioning agents conforming to the formula: 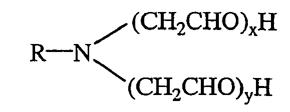 wherein R is an alkyl, preferably having from 8 to 30 carbon atoms, and x and y are integers, preferably wherein x+y is in the range of from 1 to 100, more preferably 50, event more preferably of between 1 to 20.

### Oxidizing agents

The compositions according to the present invention comprise or are used in combination with a composition that comprises at least one oxidizing agent. Preferred oxidizing agents for use herein are water-soluble peroxygen oxidizing agents. "Water-soluble" as defined herein means that in standard condition at least 0.1 g, preferably 1 g, more preferably 10g of said oxidizing agent can be dissolved in 1 liter of deionized water. The oxidizing agents are valuable for the initial solubilisation and decolorisation of the melanin (bleaching) and accelerate the polymerization of the oxidative dye precursors (oxidative dyeing) in the hair shaft.

Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Mixtures of two or more such oxidizing agents can be used if desired. Preferred for use in the compositions according to the present invention is hydrogen peroxide.

In conventional dyeing and bleaching compositions, levels of peroxygen oxidizing agent are usually of from about 0.1% to about 7% by weight. Higher levels, whilst giving good results in term of efficacy were until now not practical because of increased hair damage. The oxidative damage protection provided by the present invention makes it now possible to use oxidizing agent such as hydrogen peroxide in level up to 40% in the oxidizing composition. However, for safety reasons, level above 12% should be carefully investigated before being used on human. Preferably, the level of the oxidizing agent in the oxidizing composition is of from about 0.5% to about 20% by weight, more preferably of from about 1% to about 15%. The compositions according to the present invention provide excellent gray coverage, vibrant colors and acceptable damage at level above about 7% (typically about 12%).

### Additional components

Moreover, it is also intended that the compositions of the present invention may be complex compositions, which in addition to the chelant and oxidizing agent comprise other components that may or may not be active ingredients. This includes, but is not limited to, buffering agents, hair dyeing agents such as oxidative dye precursors, non-oxidative dyes, thickeners, solvents, enzymes, anionic, non ionic, amphoteric and cationic surfactants, carriers, antioxidants, stabilizers, perming actives, perfume, hair swelling agents and/or polymers. Some of these additional components are detailed hereafter.

Finally, the compositions according to the present invention can be provided in any usual form, such as for example an aqueous composition, a powder, a gel or an oil-in-water emulsion. Preferred media for the compositions according to the present invention are thickened aqueous solutions comprising a salt-tolerant thickener or oil-in-water emulsions.

### pH buffering agents

The compositions according to the present invention preferably further comprise a pH buffering agent. The pH of the composition is preferably of from about 8 to about 12, more preferably of from about 9 to about 11, even more preferably of from about 9.5 to about 10.5. Suitable buffering agents are well known in the art and include for example ammonia/ammonium acetate mixture and monoethanolamine (MEA).

### Oxidative hair dye precursors

These compounds are well known in the art, and include aromatic diamines, aminophenols and their derivatives (a representative but not exhaustive list of oxidation dye precursor can be found in Sagarin, "Cosmetic Science and Technology", "Interscience, Special Edn. Vol. 2 pages 308 to 310). Precursors can be used with couplers. Couplers are genreally colorless molecules that can form colors in the presence of activated precursors.

The choice of precursors and couplers will be determined by the color, shade and intensity of coloration that is desired. The precursors and couplers can be used herein, singly or in combination, to provide dyes having a variety of shades ranging from ash blonde to black.

Hair dye compositions will generally comprise from about 0.001 % to about 10%, preferably from about 0.1% to about 2%, of oxidative dye precursors and couplers.

### Thickeners

The composition of the present invention may optionally further comprise at least about 0.1% of thickeners. Thickeners are preferably comprised in amount sufficient to provide the composition with a viscosity of from about 1 Pa.s to 10 Pa.s (1,000 to 10,000 cP) at 26°Cin order to provide a composition that can be readily applied to the hair without dripping.

Preferred for use herein are salt tolerant thickeners. Salt-tolerant thickeners are functionally defined herein as compounds that increases the viscosity of an aqueous composition consisting of 3.8% DTPMP (tetrasodium salt) and 1.95% NH₃ to at least 1 Pa.s (1,000 cP) when incorporated at levels of 2% by weight as measured at 26.7°C. The viscosity can be measured with a Brookfield viscometer DVII, using S41 spindles for samples under 10 Pa.s (10,000 cP) and spindle S52 for samples over 10 Pa.s (10,000 cP) (available from Brookfield), with a speed rating of 1 revolution per minute and samples sizes of 2ml (for S41 spindle) or 0.5 ml (for S52 spindle).

A non exclusive list of suitable salt tolerant thickeners for use herein include xanthan, guar, hydroxypropyl guar, scleroglucan, methyl cellulose, ethyl cellulose (commercially available as Aquacote®), hydroxyethyl cellulose (Natrosol®), carboxymethyl cellulose, hydroxypropylmethyl cellulose, microcrystalline cellulose, hydroxybutylmethyl cellulose, hydroxypropyl cellulose (Klucel®), hydroxyethyl ethyl cellulose, cetyl hydroxyethyl cellulose (NatrosolⓇ Plus 330), N-vinylpyrollidone (Povidone®), Acrylates / Ceteth-20 Itaconate Copolymer (Structure® 3001), hydroxypropyl starch phosphate (Structure® ZEA), polyethoxylated urethanes or polycarbamyl polyglycol ester (e.g. PEG-150/DecyUSMDI copolymer = Aculyn® 44, PEG-150/Stearyl/SMDI copolymer = Aculyn 46®), trihydroxystearin (Thixcin®) acrylates copolymer (e.g. Aculyn® 33) or hydrophobically modified acrylate copolymers (e.g. Acrylates / Steareth-20 Methacrylate Copolymer = Aculyn® 22).

Fatty alcohols have thickening properties and can be used in the compositions present invention. Fatty alcohols are however not salt-tolerant thickeners according to the above definition. A mixture of 2% cetyl and stearyl alcohol has for example a viscosity of less than about 0.7 Pa.s (700 cP) as measured at 26°C with a Brookfield viscometer in the conditions disclosed hereabove.

### EXPERIMENTALS

### Total combing force

The following illustrates the effect of chelants on the deposition of an amino silicone and dicetyldimonium chloride conditioner.

A standard bleach composition comprising 1.95% NH₃ and 4.5% hydrogen peroxide was prepared and was used as reference. Three other bleach compositions were prepared using portions of this standard bleach composition. To the first was added 1% by weight EDDS (trisodium salt), to the second 2.25% of a mixture of amino silicone and dicetyldimonium chloride conditioning agents, and to the third 1% EDDS and 2.25% of the same mixture of conditioning agents. Switches of virgin hair were independently bleached once using these four compositions and shampooed 10 times with a standard shampoo comprising no conditioners. The total combing force in mJ was then measured for each switch as summarized below:

| Test Product | Total combing force in mJ |
|---|---|
| Standard bleach | > 400* |
| Standard bleach + 1% EDDS | > 400 |
| Standard bleach + 2.25% conditioners | 168 |
| Standard bleach + 1% EDDS + 2.25% conditioners | 123 |

| | |
|---|---|
| * >400 indicates that the switch were not combable. | |

The total combing force is measured by passing a comb through a switch of wet hair and recording the total energy consumed. In this experiment the machine used was an Instron 5500 Tensile tester fitted with 10N static load cell. The hair switches (6 grams, 10 inch long) were fitted to the load cell and a comb was fixed to the instrument stand.

The total combing force is commonly used in the industry to quantify the quality of hair. With this setting, a total coming force of less than 150 mJ indicates hair that are easily combable and with excellent feel.

### Deposition test

The following illustrates the effect of chelants on the deposition of a typical alkoxylated amine conditioning agent: PEG2-soyamine. Three bleaching compositions comprising 1.95% NH₃, 4.5% hydrogen peroxide, 0.23% PEG2-soyamine and, respectively 0, 1.2 and 5% EDDS (trisodium salt) were prepared. Virgin hair switches were separately bleached once using these compositions and the amount of the amount of PEG-2 soyamine deposited on hair for each switch was measured* after rinsing. The values obtained were as follows:

| Test Product | Amount of PEG2-Soyamine deposited (mg deposited/g of hair) |
|---|---|
| Standard bleach + 1% PEG-2 Soyamine | 0.02 |
| Standard bleach + 1% PEG-2 Soyamine + 1.2% EDDS | 0.16 |
| Standard bleach + 1% PEG-2 Soyamine + 5% EDDS | 0.51 |

| | |
|---|---|
| * The analytical method is as follows: • Extraction of PEG-2 soyamine. 1g of hair is immersed in 10ml of dicloromethane. The sample is shaken mechanically for one hour. • The extract is then pipetted into a 2ml chromatography vial. • Analysis of PEG-2 soyamine is performed using High Performance Liquid Chromatography with Light Scattering Detection. The mobile phase used is 0.0075M ammonium formate in methanol. • Calibration, linearity, accuracy and precision is performed using a PEG-2 soyamine standard. | |

### EXAMPLES

The following examples illustrate oxidative dye compositions according to the present invention. It is understood that the examples and embodiments described herein are for illustrative purposes only and that various modifications or changes in light thereof will be suggested to one skilled in the art without departing from the scope of the present invention.

### Examples of formulation: emulsion

| | **1** | **2** | **3** | **4** | **5** | **6** | **7** | **8** | **9** | **10** |
|---|---|---|---|---|---|---|---|---|---|---|
| Sodium sulphite | 0.1 | 0.1 | 0.1 | 0.1 | 0.1 | 0.1 | 0.1 | 0.1 | 0.1 | 0.1 |
| Ascorbic Acid | 0.1 | 0.1 | 0.1 | 0.1 | 0.1 | 0.1 | 0.1 | 0.1 | 0.1 | 0.1 |
| Ammonium Acetate | 1.5 | 1.5 | 1.5 | 1.5 | 1.5 | 1.5 | 1.5 | 1.5 | 1.5 | 1.5 |
| Ammonia (30% active) | 6.0 | 6.0 | 6.0 | 6.0 | 6.0 | 6.0 | 6.0 | 6.0 | 6.0 | 6.0 |
| Ceteareth 25 | 1.0 | 1.0 | 1.0 | 1.0 | 1.0 | 1.0 | 1.0 | 1.0 | 1.0 | 1.0 |
| Cetyl Alcohol | 1.6 | 1.6 | 1.6 | 1.6 | 1.6 | 1.6 | 1.6 | 1.6 | 1.6 | 1.6 |
| Stearyl Alcohol | 3.3 | 3.3 | 3.3 | 3.3 | 3.3 | 3.3 | 3.3 | 3.3 | 3.3 | 3.3 |
| Sodium Benzoate | 0.1 | 0.1 | 0.1 | 0.1 | 0.1 | 0.1 | 0.1 | 0.1 | 0.1 | 0.1 |
| Phenoxyethanol | 0.1 | 0.1 | 0.1 | 0.1 | 0.1 | 0.1 | 0.1 | 0.1 | 0.1 | 0.1 |
| HEDP (disodium salt of) | 2.5 | - | 3.5 | - | 2.5 | 1.0 | - | 1.0 | 0.5 | 5.0 |
| EDTA (tetrasodium salt of) | - | 1.0 | 0.5 | 2.0 | - | - | 0.6 | - | 1.5 | - |
| EDDS (trisodium salt of) | - | 1.0 | - | 2.0 | 0.5 | 1.0 | 1.0 | 1.0 | 1.0 | - |
| Para-phenylene diamir | 0.8 | 0.5 | 0.6 | 0.5 | 0.8 | 0.8 | 0.5 | 0.6 | 0.5 | 0.8 |
| Para-aminophenol | 0.2 | 0.3 | 0.2 | 0.1 | 0.2 | 0.2 | 0.3 | 0.2 | 0.1 | 0.2 |
| Meta-aminophenol | 1.0 | 0.5 | 1.0 | 0.6 | 1.0 | 1.0 | 0.5 | 1.0 | 0.6 | 1.0 |
| Resorcinol | 1.6 | 1.2 | 1.6 | 0.8 | 1.6 | 1.6 | 1.2 | 1.6 | 0.8 | 1.6 |
| Hydrogen Peroxide (35% active) | 8.6 | 8.6 | 8.6 | 13 | 17 | 17 | 17 | 34 | 34 | 34 |
| Trimethylsilylamodimethicone (SF1708) | 0.5 | 0.5 | 1.5 | 2.0 | 2.0 | 2.0 | 2.0 | 2.0 | 2.0 | 2.0 |
| Polyquaternium 10 (Polymer JR30M) | 0.2 | 0.2 | - | - | 0.2 | 0.2 | 0.2 | | - | - |
| Xanthan gum | 0.5 | 0.5 | - | 1.0 | 0.5 | 0.5 | 0.5 | 0.5 | 0.5 | 1.0 |
| Cetyl hydroxyethyl Cellulose (Natrosol 330CS Plus) | - | - | 0.8 | 1.0 | 0.8 | 0.5 | 0.5 | 0.5 | 0.5 | 1.0 |
| pH adjust to pH 10 | qs | qs | qs | qs | qs | qs | qs | qs | qs | qs |
| Water | qs | qs | qs | qs | qs | qs | qs | qs | qs | qs |

### Example of formulation: thickened aqueous solution

| | **1** | **2** | **3** | **4** | **5** | **6** | **7** | **8** | **9** | **10** |
|---|---|---|---|---|---|---|---|---|---|---|
| Sodium sulphite | 0.1 | 0.1 | 0.1 | 0.1 | 0.1 | 0.1 | 0.1 | 0.1 | 0.1 | 0.1 |
| Ascorbic Acid | 0.1 | 0.1 | 0.1 | 0.1 | 0.1 | 0.1 | 0.1 | 0.1 | 0.1 | 0.1 |
| Citric Acid | 1.0 | 1.0 | 1.0 | 1.0 | 1.0 | 1.0 | 1.0 | 1.0 | 1.0 | 1.0 |
| Ammonia (30% active) | 6.0 | 6.0 | 6.0 | 6.0 | 6.0 | 6.0 | 6.0 | 6.0 | 6.0 | 6.0 |
| Acrylates Copolymer (Aculyn® 33A) | 2.4 | 2.4 | 2.4 | 2.4 | 2.4 | 2.4 | 2.4 | 2.4 | 2.4 | 2.4 |
| Oleth 10 | 1.0 | 1.0 | 1.0 | 1.0 | 1.0 | 1.0 | 1.0 | 1.0 | 1.0 | 1.0 |
| Oleth 2 | 0.8 | 0.8 | 0.8 | 0.8 | 0.8 | 0.8 | 0.8 | 0.8 | 0.8 | 0.8 |
| Oleic Acid | 0.9 | 0.9 | 0.9 | 0.9 | 0.9 | 0.9 | 0.9 | 0.9 | 0.9 | 0.9 |
| Cocamide DEA | 3.0 | 3.0 | 3.0 | 3.0 | 3.0 | 3.0 | 3.0 | 3.0 | 3.0 | 3.0 |
| HEDP (disodium salt of) | 2.5 | - | 3.5 | - | 2.5 | 1.0 | - | 1.0 | 0.5 | 5.0 |
| EDTA (tetrasodium salt of) | - | 1.0 | 0.5 | 2.0 | - | - | 0.6 | - | 1.5 | - |
| EDDS (trisodium salt of) | - | 1.0 | - | 2.0 | 0.5 | 1.0 | 1.0 | 1.0 | 1.0 | - |
| Para-phenylene diamine | 0.8 | 0.5 | 0.6 | 0.5 | 0.8 | 0.8 | 0.5 | 0.6 | 0.5 | 0.8 |
| Para-aminophenol | 0.2 | 0.3 | 0.2 | 0.1 | 0.2 | 0.2 | 0.3 | 0.2 | 0.1 | 0.2 |
| Meta-aminophenol | 1.0 | 0.5 | 1.0 | 0.6 | 1.0 | 1.0 | 0.5 | 1.0 | 0.6 | 1.0 |
| Resorcinol | 1.6 | 1.2 | 1.6 | 0.8 | 1.6 | 1.6 | 1.2 | 1.6 | 0.8 | 1.6 |
| Hydrogen Peroxide (35% active) | 8.6 | 8.6 | 8.6 | 13 | 17 | 17 | 17 | 34 | 34 | 34 |
| Behentrimonium Chloride | 0.5 | 0.5 | 1.5 | 2.0 | 2.0 | 2.0 | 2.0 | 2.0 | 2.0 | 2.0 |
| Dicetyldimonium Chloride | 0.2 | 0.2 | 0.7 | 0.2 | 0.2 | 0.2 | 0.2 | - | - | - |
| Acrylates Steareth-20 Methacrylate Copolymer (Aculyn ® 22) | 0.5 | 0.5 | - | 1.0 | 0.5 | 0.5 | 0.5 | 0.5 | 0.5 | 1.0 |
| Propylene Glycol | 8.2 | 8.2 | 8.2 | 8.2 | 8.2 | 8.2 | 8.2 | 8.2 | 8.2 | 8.2 |
| Ethoxy Diglycol | 4.2 | 4.2 | 4.2 | 4.2 | 4.2 | 4.2 | 4.2 | 4.2 | 4.2 | 4.2 |
| pH adjust to pH 10 | qs | qs | qs | qs | qs | qs | qs | qs | qs | qs |
| Water | qs | qs | qs | qs | qs | qs | qs | qs | qs | qs |

The above compositions are useful for dyeing hair with reduced damage and improved conditioner deposition. Similar compositions not including oxidative dye precursors and couplers (in the above examples para-aminophenol, meta-aminophenol and resorcinol) can be used for bleaching (lightening) hair.

Oxidative hair dye compositions are usually sold in kits comprising, in separate containers, a dye component (also called "dye cream" for emulsion or "dye liquid" for solution) comprising the oxidative dye precursors (and usually the Hair Swelling Agent) and a hydrogen peroxide component (also called "hydrogen peroxide cream" for emulsion or "hydrogen peroxide liquid" for solution) comprising the oxidizing agent (usually hydrogen peroxide). The consumer mixes the dye component and hydrogen peroxide component immediately before use. The examples of the tables above illustrate the resulting mixtures.

Similarly, bleaching compositions are usually sold as a kit comprising two or three separate containers. The first contains the hair-swelling agent (e.g. ammonia), the second contains the oxidizing agent and the third (optional) contains a second oxidizing agent (e.g. alkali or ammonium salt of persulphates, percarbonate, perborate). The bleaching compositions are obtained by mixing the above-mentioned compositions immediately before use.

These kits are well known in the art and the composition in each container can be manufactured utilizing any one of the standard approaches, these include:
- Oil in water process
- Phase Inversion process
- One-pot process

The chelants are usually added to a proportion of the water at the start of the making process at ambient temperature, and allowed to dissolve. The fatty components are then added and the mixture is processed as is normal for the above outlined procedures. For example, in a 1 pot process the polymers and chelants would be predissolved in water, the fatty materials added and then the whole heated to about 70-80°C.

A controlled cooling and optional shearing process to form the final structured product in the case of an emulsion would then follow. Addition of the ammonia and pH trimming complete the making process of the dye cream.

In the case of a liquid solution comprising acrylate polymers, these would be formulated into the hydrogen peroxide component. The glycol solvents and fatty components are formulated into the dye component. A structured product is formed when the dye and hydrogen peroxide components are mixed together prior to use of the composition, through deprotonation of the polymer acrylic acid groups yielding a polymeric micro-gel. Further details on the manufacture of these two-part aqueous composition for coloring hair, which forms a gel on mixing of the two parts can be found in US 5,376,146, Casperson et al. and US5,393,305, Cohen et al.

The composition of the present invention can also be formulated as 2-part aqueous compositions comprising polyetherpolyurethane as thickening agent (such as Aculyn ® 46) as described in US 6,156,076, Casperson et al. and US6,106,578, Jones.

When the compositions of different containers are mixed before use and the resulting mixture comprises the chelants and conditioning agents claimed, there is no preference on how the chelants and conditioning agents are distributed in these containers. Obviously chelants that can be altered by hydrogen peroxide (or any oxidizing agent used) such as secondary amine chelants should however be formulated in the dye component. The hydrogen peroxide component should however preferably comprise at least about 0.1 % of a stable chelant to stabilize hydrogen peroxide. This stabilizer is required to prevent the hydrogen peroxide from decomposing too rapidly. For example EDTA can be used in the hydrogen peroxide component as stabilizer.

### METHODS OF USE

It is understood that the examples of methods of use and embodiments described herein are for illustrative purposes only and that various modifications or changes in light thereof will be suggested to one skilled in the art without departing from the scope of the present invention.

### Without pretreatment

The chelants according to the present invention are preferably formulated directly in the oxidizing compositions applied on hair (e.g. oxidative dye composition or bleach).

### Oxidative dye

Oxidative dye compositions are usually sold as a kit comprising at least two separate containers: one contains the oxidative dye precursors with the hair-swelling agent (e.g. ammonia) in a suitable carrier (e.g. dye cream or liquid) and the other contains the oxidizing agent in a suitable carrier (e.g. hydrogen peroxide cream or liquid). The consumer prepares the oxidative dye composition immediately before use by mixing both compositions and applies it on hair. After working the mixture a few minutes (to insure uniform application to all of the hair), the oxidative dye composition is allowed to remain on the hair for an amount sufficient for the dyeing to take place (usually about 30 minutes). The consumer then rinses his/her hair thoroughly with tap water and allows it to dry. It is observed that the hair has changed from its original color to the desired color.

The conditioning agent can also be packaged partly or in totality in a third container. In this case, all three compositions can be mixed immediately before use and applied together, or the content of the third container can be applied (with an optional rinse step) as post-treatment immediately after the oxidative dye composition resulting from the mixture of the other containers.

### Bleaching compositions

Bleaching compositions are usually sold as a kit comprising two or three separate containers. The first contains the hair-swelling agent (e.g. ammonia), the second contains the oxidizing agent and the third (optional) contains a second oxidizing agent (e.g. alkali or ammonium salt of persulphates, percarbonate, perborate). The consumer prepares the bleaching compositions immediately before use by mixing all compositions and applies the mixture on hair (as for the oxidative dye composition) for an amount of time sufficient for the bleaching to take place (usually about 30mn).

The conditioning agent can also be packaged partly or in totality in an additional third or fourth container. In this case, all compositions can be mixed together immediately before use or the content of the additional container can be applied (with an optional preliminary rinse step) as a post-treatment immediately after the bleaching composition resulting from the mixture of the other containers.

### With pretreatment

The chelants and conditioners can also be applied to hair as a pretreatment. The pretreatment composition ("first composition") can be applied immediately before the oxidizing composition ("second composition") or after a longer period of time.

### Pretreatment immediately followed by an oxidizing composition

In the case of a pretreatment applied on hair and immediately followed by the oxidizing composition, said pretreatment composition can be rinsed off hair before the application of the oxidizing composition, but will be preferably kept on the hair during the application of the oxidizing compositions, the resulting mixture being rinsed off following the oxidizing step. Kits comprising one container for the first composition (pre-treat) and one, two or more containers for the second composition (oxidizing composition) can be advantageously used for this method. Two containers or more can be required for the second composition in case this second composition is prepared immediately before use by mixing the content of two containers or more (e.g. oxidative hair dye composition or bleaching composition). The kit can also comprise an additional container for a composition comprising a conditioning agent that is applied independently from the second composition in a third step, optionally following a rinsing step.

### Color care

The pretreatment can also take place as a "color care" treatment anytime between two oxidative treatments but not immediately prior to one. The 2 oxidative treatments are preferably separated by at least one day, more preferably at least one week. Oxidative hair dye treatments are generally repeated about once a month and obviously, hair will be usually rinsed with water immediately after each oxidative treatment. The "color care" treatment can be repeated as many times as practical between the two oxidative treatments, which can be once, twice or more.

## Claims

1. A hair treatment composition comprising:
a) an oxidizing agent;
b) a conditioning agent selected from silicone materials, especially nonvolatile silicones and amino functionalised silicones, cationic surfactants, cationic polymers, alkoxylated amines and mixtures thereof; and at least one oxidative hair dye precursor,
**characterized in that** said composition further comprises:
c) at least 1.6%, by weight of the composition, of a chelant.

2. A composition according to Claim 1 wherein the chelant is selected from:
i) carboxylic acid, in particular aminocarboxylic acid, chelants;
ii) phosphonic acid, in particular aminophosphonic acid, chelants;
iii) polyphosphoric acid, in particular linear polyphosphonic, chelants; and
iv) salts thereof, and mixtures thereof.

3. A composition according to Claim 2, wherein said chelant is selected from aminocarboxylic acid chelants selected from the group consisting of diamine-N,N'-dipolyacids, monoamine monoamide-N,N'-dipolyacids and N,N'-bis(2-hydroxybenzyl)ethylenediamine-N,N'-diacetic acid (HBED), salts thereof, and mixtures thereof.

4. A composition according to Claim 3 wherein said polyacids contain at least two acid groups independently selected from the carboxylic acid group (-COOH), sulphonic group (-SO₃H), the o-hydroxyphenyl group, the m-hydroxyphenyl group and the p-hydroxyphenyl group.

5. A composition according to Claim 4 wherein said chelant is selected from the group consisting of ethylenediamine-N,N'-disuccinic acid (EDDS), ethylenediamine-N,N'-diglutaric acid (EDDG), 2-hydroxypropylenediamine-N,N'-disuccinic acid (HPDDS), glycinamide-N,N'-disuccinic acid (GADS), ethylenediamine-N-N'-bis(ortho-hydroxyphenyl acetic acid) (EDDHA), salts thereof, and mixtures thereof.

6. A composition according to any of the preceding Claims wherein the composition is in the form of an oil-in-water emulsion or a thickened aqueous solution.

7. A composition according to any of the preceding Claims further comprising a salt-tolerant thickener, said tolerant thickener being preferably selected from xanthan, guar, hydroxypropyl guar, scleroglucan, methyl cellulose, ethyl cellulose, hydroxyethyl cellulose, carboxymethyl cellulose, hydroxypropylmethyl cellulose, microcrystalline cellulose, hydroxybutylmethyl cellulose, hydroxypropyl cellulose, hydroxyethyl ethyl cellulose, cetyl hydroxyethyl cellulose, N-vinylpyrollidone, Acrylates / Ceteth-20 Itaconate Copolymer, hydroxypropyl starch phosphate, polyethoxylated urethanes or polycarbamyl polyglycol ester trihydroxystearin, acrylates copolymer or hydrophobically modified acrylate copolymers and mixtures thereof.

8. A composition according to any of the preceding Claims wherein the pH is above 8, preferably between 8 and 12, more preferably between 9 and 11.5, even more preferably between 9.5 and 11.

9. A composition according to any of the preceding Claims wherein said conditioning agent is selected from silicone materials.

10. A method of treating hair comprising the step of contacting hair with a composition according to any of the preceding Claims.

11. A method of treating hair comprising the subsequent steps of:
i) contacting hair with a first composition comprising an oxidizing agent, at least 1.6% by weight of said first composition of chelant; and at least one oxidative dye precursor; and
ii) contacting hair immediately after step i) with a second composition comprising a conditioning agent, selected from silicone materials, especially non-volatile silicones and amino functionalised silicones, cationic surfactants, cationic polymers, alkoxylated amines and mixtures thereof.

12. A method of treating hair according to Claim 11 wherein said first composition is rinsed off hair before step ii) and comprises, by weight of said first composition, at least 2% of said chelant.

13. A kit for dyeing hair comprising a first and a second compositions packaged in different containers, wherein said first composition comprises an oxidizing agent and said second composition comprises an hair-swelling agent and an oxidative dye precursor, **characterized in that** the resulting mixture of said first and second compositions is a composition according to Claim 1.

## Patentansprüche

1. Haarbehandlungszusammensetzung, die Folgendes umfasst:
a) ein Oxidationsmittel,
b) ein Konditioniermittel ausgewählt aus Siliconmaterialien, insbesondere nichtflüchtigen Siliconen und aminofunktionalisierten Silikonen, kationischen Tensiden, kationischen Polymeren, alkoxylierten Aminen und Mischungen davon, und mindestens einen oxidativen Haarfarbstoffvorläufer,
**dadurch gekennzeichnet, dass** die Zusammensetzung außerdem Folgendes umfasst:
c) zu mindestens 1,6 Gew.-% der Zusammensetzung einen Chelatbildner.

2. Zusammensetzung nach Anspruch 1, wobei der Chelatbildner ausgewählt ist aus Folgendem:
I) Carbonsäure-Chelatbildnern, insbesondere Amincarbonsäure-Chelatbildnern,
II) Phosphonsäure-Chelatbildnern, insbesondere Aminphosphonsäure-Chelatbildnern,
III) Polyphosphorsäure-Chelatbildnern, insbesondere linearen Polyphosphorsäure-Chelatbildnern, und
IV) Salzen davon und Mischungen davon.

3. Zusammensetzung nach Anspruch 2, wobei der Chelatbildner ausgewählt ist aus Amincarbonsäure-Chelatbildnern ausgewählt aus der Gruppe bestehend aus Diamin-N,N'-dipolysäuren, Monoaminmonoamid-N,N'-dipolysäuren und N,N'-Bis(2-hydroxybenzyl)ethylendiamin-N,N'-Acetessigsäure (HBED), Salzen davon und Mischungen davon.

4. Zusammensetzung nach Anspruch 3, wobei die Polysäuren mindestens zwei Säuregruppen enthalten, die unabhängig voneinander ausgewählt sind aus der Carbonsäuregruppe (-COOH), der Sulfongruppe (-SO₃H), der o-Hydroxyphenylgruppe, der m-Hydroxyphenylgruppe und der p-Hydroxyphenylgruppe.

5. Zusammensetzung nach Anspruch 4, wobei der Chelatbildner ausgewählt ist aus der Gruppe bestehend aus Ethylendiamin-N,N'-Dibernsteinsäure (EDDS), Ethylendiamin-N,N'-Diglutarsäure (EDDG), 2-Hydroxypropylendiamin-N,N'-Dibernsteinsäure (HPDDS), Glycinamid-N,N'-Dibernsteinsäure (GADS), Ethylendiamin-N-N'-bis(ortho-hydroxyphenylessigsäure) (EDDHA), Salzen davon und Mischungen davon.

6. Zusammensetzung nach einem der vorstehenden Ansprüche, wobei die Zusammensetzung in Form einer Öl-in-Wasser-Emulsion oder einer verdickten wässrigen Lösung vorliegt.

7. Zusammensetzung nach einem der vorstehenden Ansprüche, die ferner ein salztolerantes Verdickungsmittel umfasst, wobei das salztolerante Verdickungsmittel vorzugsweise ausgewählt ist aus Xanthan, Guargummi, Hydroxypropyl-Guargummi, Scleroglucan, Methylcellulose, Ethylcellulose, Hydroxyethylcellulose, Carboxymethylcellulose, Hydroxypropylmethylcellulose, Microcrystallincellulose, Hydroxybutylmethylcellulose, Hydroxypropylcellulose, Hydroxyethylethylcellulose, Cetylhydroxyethylcellulose, N-Vinylpyrollidon, Acrylat- / Ceteth-20 Itaconat-Copolymer, Hydroxypropyl-Stärkephosphat, polyethoxylierten Urethanen oder Polycarbamylpolyglycolestern, Trihydroxystearin, Acrylatcopolymer oder hydrophobisch modifizierten Acrylatcopolymeren und Mischungen davon.

8. Zusammensetzung nach einem der vorstehenden Ansprüche, wobei der pH-Wert über 8 beträgt, vorzugsweise zwischen 8 und 12, bevorzugter zwischen 9 und 11,5, noch bevorzugter zwischen 9,5 und 11.

9. Zusammensetzung nach einem der vorstehenden Ansprüche, wobei das Konditioniermittel ausgewählt ist aus Siliconmaterialien.

10. Verfahren zur Haarbehandlung, das den Schritt des Inkontaktbringens des Haars mit einer Zusammensetzung gemäß einem der vorstehenden Ansprüche umfasst.

11. Verfahren zur Haarbehandlung, das nacheinander folgende Schritte umfasst:
I) Inkontaktbringen des Haars mit einer ersten Zusammensetzung, die ein Oxidationsmittel umfasst, zu mindestens 1,6 Gew.-% die erste Chelatbildner-Zusammensetzung und mindestens einen oxidativen Farbstoffvorläufer, und
II) Inkontaktbringen des Haars mit einer zweiten Zusammensetzung, die ein Konditioniermittel ausgewählt aus Siliconmaterialien, insbesondere nichtflüchtigen Siliconen und aminofunktionalisierten Siliconen, kationischen Tensiden, kationischen Polymeren, alkoxylierten Aminen und Mischungen davon umfasst, und zwar unmittelbar nach Schritt I).

12. Verfahren zur Haarbehandlung nach Anspruch 11, wobei die erste Zusammensetzung vor Schritt II) aus dem Haar ausgespült wird und, bezogen auf das Gewicht der ersten Zusammensetzung, zu mindestens 2 % den Chelatbildner umfasst.

13. Haarfärbeset, das eine erste und eine zweite Zusammensetzung umfasst, die in verschiedenen Behältern verpackt sind, wobei die erste Zusammensetzung ein Oxidationsmittel umfasst und die zweite Zusammensetzung ein Haarquellmittel und einen oxidativen Farbstoffvorläufer umfasst, **dadurch gekennzeichnet, dass** die resultierende Mischung aus der ersten und der zweiten Zusammensetzung eine Zusammensetzung nach Anspruch 1 ist.

## Revendications

1. Composition de traitement capillaire comprenant :
a) un agent oxydant ;
b) un agent de conditionnement choisi parmi les matériaux siliconés, spécialement des silicones non volatiles et des silicones amino fonctionnalisées, des agents tensioactifs cationiques, des polymères cationiques, des amines alcoxylées et leurs mélanges ; et au moins un précurseur de teinture capillaire oxydative,
**caractérisée en ce que** ladite composition comprend, en outre :
c) au moins 1,6 % en poids de la composition, d'un agent chélatant.

2. Composition selon la revendication 1, dans laquelle l'agent chélatant est choisi parmi :
i) des agents chélatants de type acide carboxylique, en particulier acide aminocarboxylique ;
ii) des agents chélatants de type acide phosphonique, en particulier l'aminophosphonique ;
iii) des agents chélatants de type acide polyphosphorique, en particulier polyphosphonique linéaire ; et
iv) leurs sels, et leurs mélanges.

3. Composition selon la revendication 2, dans laquelle ledit agent chélatant est choisi parmi des agents chélatants de type acide aminocarboxylique choisis dans le groupe constitué de diamine-N,N'-dipolyacides, monoamine monoamide-N,N'-dipolyacides et acide N,N'-bis(2-hydroxybenzyl)éthylène-diamine-N,N'-diacétique (HBED), leurs sels, et leurs mélanges.

4. Composition selon la revendication 3, dans laquelle lesdits polyacides contiennent au moins deux groupes acides indépendamment choisis parmi le groupe acide carboxylique (-COOH), le groupe sulfonique (-SO₃H), le groupe o-hydroxyphényle, le groupe m-hydroxyphényle et le groupe p-hydroxyphényle.

5. Composition selon la revendication 4, dans laquelle ledit agent chélatant est choisi dans le groupe constitué de l'acide éthylène-diamine-N,N'-disuccinique (EDDS), l'acide éthylène-diamine-N,N'-diglutarique (EDDG), l'acide 2-hydroxypropylènediamine-N,N'-disuccinique (HPDDS), l'acide glycinamide-N,N'-disuccinique (GADS), l'acide éthylène-diamine-N-N'-bis(orthohydroxyphényle acétique) (EDDHA), leurs sels, et leurs mélanges.

6. Composition selon l'une quelconque des revendications précédentes, où la composition est sous la forme d'une émulsion huile-dans-eau ou d'une solution aqueuse épaissie.

7. Composition selon l'une quelconque des revendications précédentes, comprenant en outre un épaississant tolérant aux sels, ledit épaississant tolérant étant de préférence choisi parmi le xanthane, la gomme guar, l'hydroxypropyl guar, la scléroglucane, la méthylcellulose, l'éthylcellulose, l'hydroxyéthylcellulose, la carboxyméthylcellulose, l'hydroxypropylméthylcellulose, la cellulose microcristalline, l'hydroxybutylméthylcellulose, l'hydroxypropylcellulose, l'hydroxyéthyl-éthylcellulose, la cétyl-hydroxyéthylcellulose, la N-vinylpyrollidone, un copolymère acrylates / céteth-20 Itaconate, un phosphate d'hydroxypropyl amidon, un ester d'uréthanes polyéthoxylés ou de polycarbamyl polyglycol, la trihydroxystéarine, un copolymère d'acrylates ou des copolymères acrylate rendus hydrophobes et leurs mélanges.

8. Composition selon l'une quelconque des revendications précédentes, dans laquelle le pH est supérieur à 8, de préférence entre 8 et 12, plus préférablement entre 9 et 11,5, encore plus préférablement entre 9,5 et 11.

9. Composition selon l'une quelconque des revendications précédentes dans laquelle ledit agent de conditionnement est choisi parmi des matériaux siliconés.

10. Procédé de traitement capillaire comprenant l'étape consistant à mettre en contact les cheveux avec une composition selon l'une quelconque des revendications précédentes.

11. Procédé de traitement capillaire comprenant les étapes ultérieures consistant à :
i) mettre en contact les cheveux avec une première composition comprenant un agent oxydant, au moins 1,6 % en poids de ladite première composition d'agent chélatant ; et au moins un précurseur de teinture oxydative ; et
ii) mettre en contact les cheveux immédiatement après l'étape i) avec une deuxième composition comprenant un agent de conditionnement, choisi parmi les matériaux siliconés, spécialement des silicones non volatiles et des silicones amino fonctionnalisées, des agents tensioactifs cationiques, des polymères cationiques, des amines alcoxylées et leurs mélanges.

12. Procédé de traitement capillaire selon la revendication 11, dans lequel ladite première composition est enlevée par rinçage des cheveux avant l'étape ii) et comprend, en poids de ladite première composition, au moins 2 % dudit agent chélatant.

13. Trousse pour la teinture des cheveux comprenant une première composition et une deuxième composition, conditionnées dans des récipients différents, dans laquelle ladite première composition comprend un agent oxydant et ladite deuxième composition comprend un agent de gonflement des cheveux et un précurseur de teinture oxydative, **caractérisée en ce que** le mélange résultant desdites première et deuxième compositions est une composition selon la revendication 1.
